# DEMANDE DE BREVET EUROPEEN

(11) **EP 3 892 309 A2**
(43) Date de publication de la demande: **13.10.2021**
(21) Numéro de dépôt: 21020198.4
(22) Date de dépôt: 12.04.2021
(51) Int. Cl.: A61L 9/12, B32B 29/00, B27K 3/18

(54) **DIFFUSEUR DE PARFUM**

(30) Priorité: 11.04.2020 FR 2003672
(71) Demandeur: Miral, 92100 Boulogne-Billancourt (FR)
(72) Inventeur: PATRY, Eléonore, 92100 Boulogne-Billancourt (FR)
(74) Mandataire: Reinhardt, Yves

(57) **Abrégé**

Le diffuseur de parfum (3) comprend une partie de diffusion (10) pour diffuser le parfum par contact avec l'air environnant. La partie de diffusion est pré-imprégnée de parfum pour diffuser subséquemment à son imprégnation le parfum contenu dans la partie de diffusion sans apport extérieur de parfum ou bien il comprend en outre une partie de trempage (13) reliée à la partie de diffusion (11) et destinée à être placée dans un réservoir à parfum (20), la partie de trempage (11) étant apte à amener le parfum par capillarité depuis le réservoir à parfum (20) à la partie de diffusion (11). Dans les deux cas, la partie de diffusion (10) comprend ou est constituée par un morceau de carton ayant un grammage supérieur ou égal à 800 g/m² et ayant plusieurs couches de papier pour lesquelles tout ou partie de la tranche est exposée librement.

## Description

La présente invention concerne un diffuseur de parfum à froid.

Dans l'art antérieur, il est classique d'utiliser des bâtonnets en rotin en tant que diffuseur de parfum. Une partie inférieure des bâtonnets est placée dans un récipient contenant une solution parfumée laquelle remonte par capillarité dans les bâtonnets et est diffusé dans l'environnement par la partie des bâtonnets exposée à l'air. Ce type de diffuseur de parfum procure une durée de diffusion du parfum importante qui est fonction du volume de parfum contenu dans le récipient.

Néanmoins, un inconvénient réside dans la variabilité de la qualité de diffusion lié aux irrégularités structurelles naturellement présentes dans le rotin, en particulier des nœuds qui peuvent éventuellement réduire ou bloquer la capillarité du bâtonnet.

En variante, il existe des bâtonnets en polyester utilisés comme diffuseur de parfum qui ne présentent pas cet inconvénient. Cependant, ceux-ci ont pour inconvénient de ne pas être réalisé dans un matériau écologique.

Dans les deux cas, le diffuseur de parfum a aussi pour inconvénient d'avoir une esthétique limitée par la forme de bâtonnets, seuls le diamètre et la longueur des bâtonnets étant susceptibles d'être variés.

Sont aussi connus dans l'art antérieur les cartes ou touches à parfum. Elles sont typiquement mises à disposition dans les boutiques de parfum à l'usage de la clientèle pour servir de support sur lequel pulvériser un parfum à tester. Une carte ou touche à parfum est généralement constituée par un morceau de papier monocouche ayant un grammage habituellement compris dans la plage allant de 110 g/m² à 450 g/m². Le papier est développé pour présenter une aptitude à recevoir et restituer les parfums, en particulier il peut inclure des fibres de coton. Le papier peut être coloré et découpé en différentes formes. Il est généralement apte à être imprimé ou embossé.

Ces cartes ou touches à parfum sont adaptées en particulier à leur usage de test en boutique ou autres usages similaires, notamment parce que la personne testant un parfum va approcher son nez de la carte ou touche à parfum immédiatement après avoir pulvérisé le parfum à tester sur celle-ci.

En revanche, les cartes ou touches à parfum ne sont pas véritablement aptes à servir de diffuseurs de parfum destinés, par exemple, à parfumer avec une intensité et une durée satisfaisante un volume d'une certaine importance tel qu'une pièce d'habitation, voire même l'habitacle d'une voiture. En effet, une carte ou touche à parfum ne s'imprègne que de peu de parfum et sèche rapidement de sorte qu'elle ne parfume que pauvrement un volume d'une certaine taille telle qu'une pièce d'habitation ou un habitacle de voiture, et, en tout état de cause, elle ne le parfume que pendant une courte durée incompatible avec celle attendue pour un diffuseur de parfum. Il est notable que dans leur usage aux fins de test de parfums dans les boutiques, l'air est avant tout parfumé par la pulvérisation elle-même du parfum, ainsi que de la volatilisation rapide du parfum qui s'était déposé par pulvérisation sur la carte ou touche à parfum, et que très peu par le relargage subséquent du parfum dont s'est imprégnée la carte ou touche à parfum.

Il est jugé depuis longtemps nécessaire de recourir à d'autres matériaux que le papier, notamment des matériaux de synthèse, et à des structures plus complexes pour réaliser des diffuseurs de parfum à froid afin de procurer à la fois une certaine intensité et durée de diffusion de parfum. Comme illustré par US 2,757, 957, US 3,065,915 et WO 2014/107562, c'est en particulier le cas des diffuseurs de parfum à froid formés par une partie de diffusion dans laquelle est incorporé le parfum en usine et qui de ce fait ne requièrent pas d'apport extérieur de parfum subséquemment lors de leur utilisation pour parfumer l'environnement.

Cependant, ces diffuseurs de parfum sont peu économiques et non écologiques, compte tenu de leur structure complexe et des matériaux employés. Le but de la présente invention est de pallier au moins partiellement les inconvénients des arts antérieurs précités.

Plus particulièrement, l'invention vise à fournir un diffuseur de parfum qui puisse procurer une diffusion de parfum satisfaisante en termes d'intensité de diffusion et de durée de diffusion, qui présente une bonne reproductibilité des caractéristiques de diffusion de parfum et qui soit susceptible d'être mis en œuvre avec une grande variété de formes de manière pouvoir varier son esthétique, tout en étant économique à fabriquer et écologique.

A cette fin, la présente invention propose un diffuseur de parfum, comprenant une partie de diffusion pour diffuser le parfum par contact avec l'air environnant. La partie de diffusion est pré-imprégnée de parfum pour diffuser subséquemment à son imprégnation le parfum contenu dans la partie de diffusion sans apport extérieur de parfum. Alternativement, le diffuseur de parfum comprend en outre une partie de trempage reliée à la partie de diffusion et destinée à être placée dans un réservoir à parfum, la partie de trempage étant apte à amener le parfum par capillarité depuis le réservoir à parfum à la partie de diffusion. Dans les deux cas, la partie de diffusion comprend ou est constituée par un morceau de carton ayant un grammage supérieur ou égal à 800 g/m² et ayant plusieurs couches de papier pour lesquelles tout ou partie de la tranche est exposée librement.

L'invention se base ainsi sur l'utilisation du papier dans le carton pour assurer une fonction de réservoir de parfum et de diffusion de celui-ci. L'invention va de ce fait à l'encontre du préjugé selon lequel il n'est pas possible de réaliser un diffuseur de parfum satisfaisant à partir de papier.

Le grammage important du carton a pour avantage d'assurer une bonne rigidité au morceau de carton, ce qui évite ou tout au moins limite sa propension à se déformer lorsqu'il est imprégné de parfum, y compris en utilisation très prolongée dans le cas où le diffuseur de parfum est pourvu d'une partie de trempage.

Un autre avantage du grammage important du carton est de rendre possible l'imprégnation du morceau de carton avec un volume de parfum augmenté. En effet, l'épaisseur du carton est corrélativement plus importante, ce qui permet de recourir à une épaisseur cumulée importante de couches imprégnables par le parfum qui sont constituées en tout ou partie par les couches de papier. Une diffusion prolongée de parfum est ainsi possible lorsqu'il s'agit d'un diffuseur de parfum pré-imprégnée qui diffuse en utilisation le parfum sans apport extérieur de parfum.

Le recours à un carton multicouche ayant plusieurs couches de papier améliore aussi la rigidité du morceau de carton par rapport au cas d'un papier ou carton monocouche ayant une épaisseur identique.

Il a aussi été constaté qu'il permet d'imprégner le morceau de carton avec une quantité plus importante de parfum par rapport au cas d'un papier ou carton monocouche ayant une épaisseur identique à l'épaisseur cumulée de la pluralité de couches de papier. Il a encore été constaté que la diffusion du parfum se faisait de façon importante, voire prépondérante, par les tranches des différentes couches de papier dès lors qu'elles étaient exposées à l'air libre et que l'intensité de la diffusion de parfum - autrement dit la quantité de parfum diffusée par unité de temps - était plus importante que pour un papier ou carton monocouche ayant une épaisseur identique à l'épaisseur cumulée de la pluralité de couches de papier.

Sans vouloir être lié par une théorie, ces avantages procurés par le fait que le morceau de carton comprenne une pluralité de couches de papier par rapport à un papier ou carton monocouche à la fois en termes de quantité de parfum pouvant imprégner le morceau de carton et d'intensité de diffusion de parfum semble résulter du fait que le parfum aurait tendance à être contenu de façon prépondérante au cœur d'une couche de papier plutôt que vers ses surfaces extérieures.

Comparé aux diffuseurs de parfum sous la forme de bâtonnets en rotin, le diffuseur de parfum de l'invention assure une bonne reproductibilité des caractéristiques de diffusion du parfum et est susceptible d'être mis en œuvre avec une plus grande variété de formes de manière à varier son esthétique.

Contrairement à une carte ou touche à parfum, le diffuseur de parfum de l'invention assure une diffusion de parfum à la fois intense et durable tout en conservant une bonne tenue.

De plus, il peut avantageusement être prévu que le morceau de carton présente un ou plusieurs ajours, tout ou partie de la tranche des couches de papier étant exposées librement dans le ou les ajours. La diffusion de parfum est de ce fait encore accrue. Comparé aux diffuseurs de parfum à froid de l'art antérieur précité qui ont une structure plus complexe à base de matériaux synthétiques, le diffuseur de parfum de l'invention est plus simple et plus économique à fabriquer et plus écologique.

Suivant des modes de réalisation préférés, le diffuseur de parfum selon l'invention comprend une ou plusieurs des caractéristiques suivantes :
- le morceau de carton est un morceau de carton compact ;
- au moins 50%, plus préférentiellement encore au moins 75%, et encore plus préférentiellement au moins 90% du grammage du morceau de carton provient de ses couches de papier ;
- les couches de papier ont chacune une longueur de tranche exposée librement d'au moins 50 cm, plus préférentiellement d'au moins 75 cm et plus préférentiellement encore d'au moins 100 cm.
- le volume des couches de papier avant imprégnation de parfum est d'au moins 5 cm3, plus préférentiellement encore d'au moins 7,5 cm³ et encore plus préférentiellement d'au moins 12,5 cm³ ;
- la partie de diffusion ou le morceau de carton de la partie de diffusion et la partie de trempage constituent un seul et même morceau de carton ;
- le morceau de carton est teinté dans la masse ;
- les couches de papier sont imprégnées ou sont aptes à être imprégnées avec du parfum.

L'invention propose aussi un ensemble comprenant un diffuseur de parfum selon l'invention tel que décrit précédemment, plus particulièrement selon l'alternative comprenant ladite partie de trempage, et un réservoir à parfum, dans lequel la partie de trempage du diffuseur de parfum est prévue pour être placée dans le réservoir à parfum pour amener par capillarité le parfum contenu dans le réservoir à la partie de diffusion placée à l'air libre.

L'invention propose encore un procédé de fabrication d'un diffuseur de parfum comprenant une partie de diffusion pour diffuser le parfum par contact avec l'air environnant, le procédé comprenant la fourniture de la partie de diffusion sous une forme qui comprend ou est constituée par un morceau de carton ayant un grammage supérieur ou égal à 800 g/m² et ayant plusieurs couches de papier pour lesquelles tout ou partie de la tranche est exposée librement.

De manière préférentielle, le morceau de carton peut en plus présenter un ou plusieurs ajours, tout ou partie de la tranche des couches de papier étant exposée librement dans le ou les ajours.

Suivant un mode de réalisation préféré, le procédé comprend l'imprégnation de la partie de diffusion avec du parfum pour diffuser subséquemment à son imprégnation le parfum contenu dans la partie de diffusion sans apport extérieur de parfum, et, après imprégnation, le conditionnement du diffuseur de parfum dans un emballage fermé hermétiquement, soit par unité, soit par lot de plusieurs exemplaires.

Suivant un autre mode de réalisation préféré, le procédé comprend la fourniture d'une partie de trempage reliée à la partie de diffusion de manière à faire partie du diffuseur de parfum, la partie de trempage étant destinée à être placée dans un réservoir à parfum et apte à amener le parfum par capillarité depuis le réservoir à parfum à la partie de diffusion. Le morceau de carton de la partie de diffusion et la partie de trempage peuvent avantageusement constitués un seul et même morceau de carton.

Suivant d'autres modes de réalisation préférés qui se combinent aussi avec les deux précédents, le procédé comprend une ou plusieurs des caractéristiques suivantes :
- le morceau de carton est un morceau de carton compact ;
- au moins 50%, plus préférentiellement au moins 75%, et plus préférentiellement encore au moins 90% du grammage du morceau de carton provient de ses couches de papier ;
- les couches de papier ont chacune une longueur de tranche exposée librement d'au moins 50 cm, plus préférentiellement d'au moins 75 cm et plus préférentiellement encore d'au moins 100 cm ;
- le volume des couches de papier avant imprégnation de parfum est d'au moins 5 cm³, plus préférentiellement d'au moins 7,5 cm³ et plus préférentiellement encore d'au moins 12,5 cm³.

D'autres aspects, caractéristiques et avantages de l'invention apparaîtront à la lecture de la description qui suit d'un mode de réalisation préféré de l'invention, donnée à titre d'exemple et en référence au dessin annexé.
[Fig 1] représente une vue de face d'un diffuseur de parfum selon un premier mode de réalisation.
[Fig 2] représente une vue de face d'un diffuseur de parfum selon un deuxième mode de réalisation.
[Fig 3] représente une vue de face d'un diffuseur de parfum selon un troisième mode de réalisation.

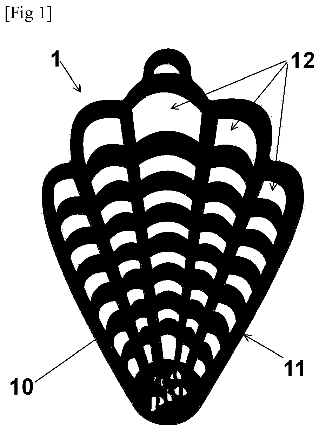

Le diffuseur de parfum 1 du premier mode de réalisation illustré par la figure 1 est uniquement constitué par une partie de diffusion 10 qui est pré-imprégnée de parfum et qui sert à diffuser le parfum après placement dans l'air environnant, par exemple dans une pièce d'un logement d'un volume pouvant aller jusqu'à 50 m³ environ ou l'habitacle d'un véhicule automobile.

Lors de la fabrication du diffuseur de parfum 1, la partie de diffusion 10 est imprégnée de parfum. Après pré-imprégnation, le diffuseur de parfum 1 - ou éventuellement plusieurs exemplaires de celui-ci - est de préférence placé dans un emballage hermétique pour préserver son imprégnation. Subséquemment, l'utilisateur extrait de son emballage le diffuseur de parfum 1 pré-imprégné de parfum et le place à l'air libre dans le lieu à parfumer. Il diffuse alors le parfum contenu dans la partie de diffusion 10. De ce fait, il n'est pas nécessaire de lui fournir un apport extérieur de parfum par exemple au moyen d'un réservoir à parfum.

Le diffuseur de parfum 1 - et donc la partie de diffusion 10 - est constitué par un morceau de carton compact. Selon un exemple non limitatif, il s'agit d'un carton ayant un grammage égal à 1900 g/m² et une épaisseur de 2,5 mm avant imprégnation, qui est constitué de cinq couches de papier contrecollées. Il s'agit en l'occurrence d'un papier 100% cellulose. Le contrecollage est réalisé au moyen d'une colle à dispersion, sans agents plastifiants, ni solvants, et en particulier par une colle d'amidon sans émission. Celui-ci présente une excellente rigidité qu'il conserve après imprégnation avec le parfum. De plus, le carton est teinté dans la masse, ce qui lui confère une belle finition, y compris après découpage et imprégnation. Il est préférable de recourir à des teintes sombres, et plus avantageusement une teinte noire, ce qui évite ou limite le risque que l'imprégnation de parfum soit visible sur la partie de diffusion 10 comme c'est le cas des cartes ou touches à parfum.

En variante, il est possible de recourir à d'autres cartons dès lors qu'ils ont un grammage d'au moins 800 g/m² et comprennent plusieurs couches de papier aptes à s'imprégner de parfum. Il est préférable néanmoins que le grammage du carton soit plus important, en particulier d'au moins 1000 g/m², voire d'au moins 1200 g/m². Similairement, il est préférable que le carton ait au moins trois couches de papier aptes à s'imprégner de parfum.

Il est préférable aussi de recourir à un carton compact plutôt qu'à un carton ondulé tant du point de vue esthétique que de la rigidité du carton après imprégnation de parfum.

Il est avantageux de recourir à un carton compact dont toutes les couches sont des couches de papier, ce qui rend le diffuseur de parfum très économique. Il n'est pas nécessaire que l'ensemble des couches de papier soient réalisées en papier 100% cellulose, bien qu'il soit préférable que chaque couche soit faite exclusivement de cellulose ou d'un mélange de cellulose et de fibres naturelles pour des raisons écologiques. Cependant, en variante, le carton peut comprendre des couches autres qu'en papier, par exemple en matériau synthétique, que ce soit en tant que couche intermédiaire ou en surface principale extérieure. Tant l'imprégnation de la partie de diffusion 10 avec du parfum que la diffusion subséquente de parfum peut le cas échéant se faire exclusivement par la tranche exposée librement des couches de papier du morceau de carton : le carton peut donc comprendre une ou plusieurs couches imperméables sans que cela soit problématique. Il est préférable cependant qu'au moins 50%, plus préférentiellement au moins 75%, et plus préférentiellement encore au moins 90% du grammage du morceau de carton provienne de ses couches de papier, ce qui permet de constituer une capacité satisfaisante de stockage de parfum dans le cas où les autres couches ne possèdent pas cette aptitude et de procurer aussi une intensité de diffusion de parfum satisfaisante. Par ailleurs, l'une des surfaces extérieures principales du carton ou les deux peuvent aussi être réalisées en papier couché.

Similairement, le contrecollage des couches de papier peut être réalisé par toute autre colle appropriée permettant de maintenir l'intégrité du collage après imprégnation.

Dans ce premier mode de réalisation, la tranche des différentes couches de papier du morceau de carton formant la partie de diffusion 10 est exposée librement sur tout le contour extérieur 11 de la partie de diffusion 10. Autrement dit, chaque couche de papier du morceau de carton est au contact par la tranche avec l'environnement extérieur sur tout le contour extérieur 11 de la partie de diffusion 10.

En variante, la tranche des différentes couches de papier peut être exposée librement sur une partie seulement du contour extérieur, par exemple dans le cas où une partie du bord du diffuseur de parfum 1 est enchâssée dans une fente d'un socle - non représenté - pour supporter le diffuseur de parfum 1 dans une position verticale. Suivant un autre exemple, une partie de la tranche du contour extérieur du morceau de carton formant la partie de diffusion 10 peut être imprimée vernie ou autrement « traitée » à des fins esthétiques, ce qui empêcherait ou limiterait la diffusion de parfum sur la partie correspondante de la tranche des couches de papier.

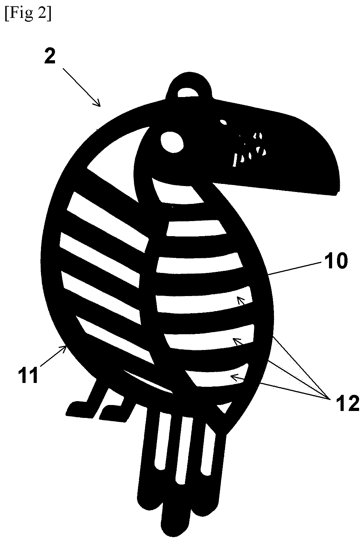

La partie de diffusion 10 peut avantageusement comprendre des ajours 12. L'on comprendra que sur la figure 1 et aussi sur la figure 2 décrite plus loin, seulement certains ajours ont été référencés par commodité, étant précisé que l'ensemble des parties représentées en noir correspond à du carton. La tranche des différentes couches de papier du morceau de carton formant la partie de diffusion 10 est également exposée librement à l'air sur toute la périphérie des ajours 12. De ce fait, la longueur de tranche exposée librement à l'air des différentes couches de papier est très significativement augmentée par rapport au seul contour extérieur 11 de la partie de diffusion 10. La diffusion de parfum est en conséquence plus intense.

En vue de parfumer une pièce ayant un volume pouvant aller jusqu'à 50 m³, il est préférable que le volume global des couches de papier du morceau de carton formant la partie de diffusion 10, avant imprégnation de parfum, soit d'au moins 5 cm³, plus préférentiellement d'au moins 7,5 cm³ et plus préférentiellement encore d'au moins 12,5 cm³. Cela permet généralement le stockage d'une quantité appropriée de parfum dans la partie de diffusion 2. Par ailleurs, la longueur de tranche exposée librement à l'air pour chacune des couches de papier est préférentiellement d'au moins 50 cm, plus préférentiellement d'au moins 75 cm et plus préférentiellement encore d'au moins 100 cm, ce qui permet généralement une diffusion suffisamment intense de parfum pour un tel volume de pièce.

La figure 2 illustre un diffuseur de parfum 2 selon un deuxième mode de réalisation. Il a la même constitution que le diffuseur de parfum 1 du premier mode de réalisation, à l'exception de la forme de son contour extérieur 11, ainsi que du nombre et de la forme des ajours 12. La description précédemment faite du diffuseur de parfum 1 selon le premier mode de réalisation est donc applicable au diffuseur de parfum 2 selon ce deuxième mode de réalisation.

Comme cela est visible de ces deux modes de réalisation, l'esthétique du diffuseur de parfum peut aisément être variée en modifiant la forme du contour extérieur, ainsi que du nombre et de la forme des ajours 12.

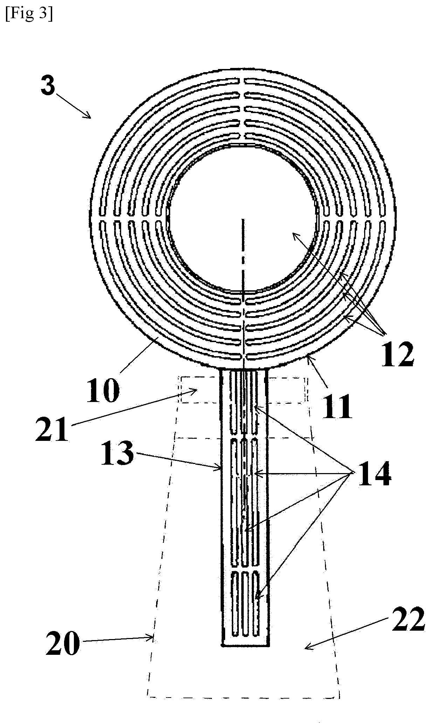

La figure 3 illustre un diffuseur de parfum 3 selon un troisième mode de réalisation. Similairement aux deux premiers modes de réalisation, il comprend une partie de diffusion 10 pour diffuser le parfum dans l'air environnant. Il comprend en outre une partie de trempage 13 reliée à la partie de diffusion 10. En utilisation du diffuseur de parfum 3, la partie de trempage 13 est placée dans un réservoir 20 contenant une solution de parfum 22 : il peut s'agir de tout flacon approprié. La partie de trempage 13 est prévue pour amener le parfum 22 par capillarité depuis le réservoir 20 à la partie de diffusion 10. La partie de diffusion 10 s'imprègne ainsi du parfum amené par la partie de trempage 13 jusqu'à la partie de diffusion 10. La partie de trempage 13 est de préférence réalisée sous la forme d'un manche, mais peut être différente notamment en considération de la forme du réservoir 20.

La constitution de la partie de diffusion 10 du diffuseur de parfum 3 est identique à celle des diffuseurs de parfum 1 et 2 des deux premiers modes de réalisation, et la description qui en a été faite s'applique de la même manière, sauf à mentionner que la pré-imprégnation avec du parfum peut être omise.

La partie de trempage 13 et la partie de diffusion 11 sont préférentiellement réalisées sous la forme d'un seul et même morceau de carton, par exemple en étant obtenu par découpage d'un seul tenant dans une plaque de carton. Cela permet d'assurer une excellente transmission du parfum par capillarité de la partie de trempage 13 à la partie de diffusion 10 du fait de la continuité de matière des différentes couches de papier formant le morceau de carton.

La partie de trempage 13 peut aussi comprendre des ajours 14 pour augmenter la longueur de tranche exposée librement des couches de papier pour favoriser l'aptitude de la partie de trempage à « aspirer » le parfum dans le réservoir 20.

Le recours dans ce troisième mode de réalisation à un carton ayant les mêmes caractéristiques que celui employé dans le cadre du premier mode de réalisation assure avantageusement à la fois une excellente rigidité dans le temps tout en assurant une diffusion intense de parfum. Il est préférable que le réservoir 20 soit pourvu d'un obturateur 21 présentant une fente de passage pour la partie de trempage 13. Cela évite l'évaporation du parfum directement du réservoir 20 dans l'environnement, c'est-à-dire dans la pièce à parfumer, et permet donc que la diffusion du parfum soit contrôlée que par le diffuseur de parfum 3 et donc essentiellement par la partie de diffusion 10. Bien entendu, l'esthétique de la partie de diffusion 10 peut également être variée en modifiant la forme de son contour extérieur 11, ainsi que la forme et le nombre d'ajours 12.

Un procédé de fabrication des diffuseurs de parfum 1, 2 et 3 des trois modes de réalisation précédemment décrits est comme suit.

La partie de diffusion 10 le cas échéant ensemble avec la partie de trempage 13, ainsi que les ajours 12, 14 sont découpés dans une plaque de carton, fournissant ainsi les diffuseurs de parfum. Les opérations de découpage peuvent avantageusement être réalisées par laser, ce qui procure à la fois une souplesse pour varier à souhait le contour extérieur et la forme des ajours, et une finition de qualité au niveau des découpes. Ensuite, au moins dans le cas des diffuseurs de parfum 1 et 2 selon les deux premiers modes de réalisation, les diffuseurs de parfum sont plongés dans une solution parfumée afin de les imprégner de parfum. La solution parfumée est préférentiellement un mélange d'alcool par exemple à 95° et de parfum concentré à 15%. Alternativement, tout liquide approprié autre que l'alcool peut être utilisé en mélange avec du parfum, par exemple du Dowanol®. La durée d'imprégnation est de préférence choisie suffisante pour saturer les différentes couches de papier du morceau de carton formant le diffuseur de parfum. Une durée de l'ordre de 3 heures est habituellement suffisante, mais pouvant aller parfois jusqu'à 12 heures.

Après retrait du bain, les diffuseurs de parfum sont soumis à une phase de séchage pour sécher leur surface extérieure et permettre à l'éventuel alcool présent de s'évaporer. Elle peut comprendre une phase d'égouttage à l'air libre dans une pièce tempérée autour de 25°C, puis éventuellement un passage dans un four pour accélérer le séchage en surface du morceau de carton. Le four est préférentiellement à une température choisie dans la plage allant de 100 à 300 °C. Le passage au four permet de raccourcir le temps de la phase de séchage. Le passage au four est optionnel, la phase de séchage pouvant être faite à l'air libre à température ambiante.

Après l'étape de séchage, les diffuseurs de parfum pré-imprégnés de parfum sont immédiatement conditionnés dans des emballages fermés hermétiquement, soit par unité, soit par lot de plusieurs exemplaires.

Bien entendu, la présente invention n'est pas limitée aux exemples et aux modes de réalisation décrits et représentés, mais elle est susceptible de nombreuses variantes accessibles à l'homme de l'art.

Par exemple, il peut être prévu que le diffuseur de parfum - qu'il soit pré-imprégné ou prévu pour le trempage dans un réservoir à parfum - comprenne plusieurs parties de diffusion 10 - et plusieurs parties de trempage 13 correspondantes le cas échéant - qui sont assemblées ou assemblables entre elles de manière à constituer un diffuseur de parfum tridimensionnel au lieu d'être plat. L'assemblage peut notamment se faire par engagement d'une partie de diffusion 10 dans une fente débouchant sur un bord extérieur d'une autre partie de diffusion 10.

Il peut aussi être prévu que la partie de diffusion 10 comprenne en plus du morceau de carton des éléments additionnels dans d'autres matériaux qui participent à la diffusion du parfum, mais le fait pour celle-ci, ainsi que de la partie de trempage 13 le cas échéant, d'être constituée uniquement par un morceau de carton a l'avantage de la simplicité et d'être économique.

Le diffuseur de parfum peut aussi comprendre des éléments additionnels par exemple en matière plastique ou des rubans de tissus à des fins décoratifs, de support ou de suspension.

## Revendications

1. Diffuseur de parfum (1, 2, 3), comprenant une partie de diffusion (10) pour diffuser le parfum par contact avec l'air environnant, dans lequel :
- la partie de diffusion est pré-imprégnée de parfum pour diffuser subséquemment à son imprégnation le parfum contenu dans la partie de diffusion sans apport extérieur de parfum, ou bien
- le diffuseur de parfum comprend en outre une partie de trempage (13) reliée à la partie de diffusion (10) et destinée à être placée dans un réservoir à parfum (20), la partie de trempage (13) étant apte à amener le parfum par capillarité depuis le réservoir à parfum (20) à la partie de diffusion (10),
dans lequel la partie de diffusion (10) comprend ou est constituée par un morceau de carton :
- ayant un grammage supérieur ou égal à 800 g/m²,
- ayant plusieurs couches de papier pour lesquelles tout ou partie de la tranche est exposée librement, et
- présentant un ou plusieurs ajours (12), tout ou partie de la tranche des couches de papier étant exposée librement dans le ou les ajours.

2. Diffuseur de parfum selon la revendication 1, dans lequel le morceau de carton est un morceau de carton compact.

3. Diffuseur de parfum selon la revendication 1 ou 2, dans lequel au moins 50%, plus préférentiellement au moins 75%, et plus préférentiellement encore au moins 90% du grammage du morceau de carton provient de ses couches de papier.

4. Diffuseur de parfum selon l'une quelconque des revendications 1 à 3, dans lequel les couches de papier ont chacune une longueur de tranche exposée librement d'au moins 50 cm, plus préférentiellement d'au moins 75 cm et plus préférentiellement encore d'au moins 100 cm.

5. Diffuseur de parfum selon l'une quelconque des revendications 1 à 4, dans lequel le volume des couches de papier avant imprégnation de parfum est d'au moins 5 cm³, plus préférentiellement d'au moins 7,5 cm³ et plus préférentiellement encore d'au moins 12,5 cm³.

6. Diffuseur de parfum selon l'une quelconque des revendications 1 à 5, dans lequel la partie de diffusion (10) ou le morceau de carton de la partie de diffusion (10) et la partie de trempage (13) constituent un seul et même morceau de carton.

7. Diffuseur de parfum selon l'une quelconque des revendications 1 à 6, dans lequel le morceau de carton est teinté dans la masse.

8. Diffuseur de parfum selon l'une quelconque des revendications 1 à 7, dans lequel les couches de papier sont imprégnées ou sont aptes à être imprégnées avec du parfum.

9. Ensemble comprenant un diffuseur de parfum (3) selon l'une quelconque des revendications 1 à 8 qui comprend ladite partie de trempage (13) et un réservoir à parfum (20), dans lequel la partie de trempage (13) du diffuseur de parfum (3) est prévue pour être placée dans le réservoir à parfum pour amener par capillarité le parfum contenu dans le réservoir (20) à la partie de diffusion (10) placée à l'air libre.

10. Procédé de fabrication d'un diffuseur de parfum (1, 2, 3) comprenant une partie de diffusion (10) pour diffuser le parfum par contact avec l'air environnant, le procédé comprenant la fourniture de la partie de diffusion (10) sous une forme qui comprend ou est constituée par un morceau de carton :
- ayant un grammage supérieur ou égal à 800 g/m²,
- ayant plusieurs couches de papier pour lesquelles tout ou partie de la tranche est exposée librement, et
- présentant un ou plusieurs ajours (12), tout ou partie de la tranche des couches de papier étant exposée librement dans le ou les ajours.

11. Procédé selon la revendication 10, comprenant en outre :
- l'imprégnation de la partie de diffusion avec du parfum pour diffuser subséquemment à son imprégnation le parfum contenu dans la partie de diffusion sans apport extérieur de parfum, et
- après imprégnation, le conditionnement du diffuseur de parfum dans un emballage fermé hermétiquement, soit par unité, soit par lot de plusieurs exemplaires.

12. Procédé selon la revendication 10, comprenant en outre la fourniture d'une partie de trempage (13) reliée à la partie de diffusion (10) de manière à faire partie du diffuseur de parfum, la partie de trempage (13) étant destinée à être placée dans un réservoir à parfum (20) et apte à amener le parfum par capillarité depuis le réservoir à parfum (20) à la partie de diffusion (10).

13. Procédé selon la revendication 12, dans lequel le morceau de carton de la partie de diffusion (10) et la partie de trempage (13) constituent un seul et même morceau de carton.

14. Procédé selon l'une quelconque des revendications 10 à 13, dans lequel le morceau de carton est un morceau de carton compact.

15. Procédé selon l'une quelconque des revendications 10 à 14, dans lequel le morceau de carton présente une ou plusieurs ou toutes les caractéristiques suivantes :
- au moins 50%, plus préférentiellement au moins 75%, et plus préférentiellement encore au moins 90% du grammage du morceau de carton provient de ses couches de papier ;
- les couches de papier ont chacune une longueur de tranche exposée librement d'au moins 50 cm, plus préférentiellement d'au moins 75 cm et plus préférentiellement encore d'au moins 100 cm ; et
- le volume des couches de papier avant imprégnation de parfum est d'au moins 5 cm³, plus préférentiellement d'au moins 7,5 cm³ et plus préférentiellement encore d'au moins 12,5 cm³.
